# EUROPEAN PATENT APPLICATION

(11) **EP 1 946 768 A1**
(43) Date of publication of application: **23.07.2008**
(21) Application number: 06822526.7
(22) Date of filing: 27.10.2006
(51) Int. Cl.: A61K 39/104, A61K 38/00, A61K 39/00, A61K 39/395, A61P 31/04, C07K 14/21, C07K 16/12, C12N 5/10, G01N 33/569, C12P 21/08

(54) **OUTER COAT PROTEIN PA5158 OF PSEUDOMONAS AERUGINOSA**

(30) Priority: 28.10.2005 JP 2005315202
(71) Applicant: Meiji Seika Kaisha, Ltd., Tokyo 104-8002 (JP)
(72) Inventor: TANAKA, Jiro, Yokohama-shi Kanagawa 222-8567 (JP); OHSAWA, Fukuichi, Yokohama-shi Kanagawa 222-8567 (JP); OKUTOMI, Takafumi, Yokohama-shi Kanagawa 222-8567 (JP); NAGASO, Hiroshi, Yokohama-shi Kanagawa 222-8567 (JP); KUMAGAI, Masashi, Yokohama-shi Kanagawa 222-8567 (JP); SUZUKI, Takahisa, Yokohama-shi Kanagawa 222-8567 (JP); OTSUKA, Keiko, Yokohama-shi Kanagawa 222-8567 (JP)
(74) Representative: Ahner, Francis
(86) International application number: PCT/JP2006/321565
(87) International publication number: WO 2007/049770

(57) **Abstract**

An object of the present invention is to provide a protein or peptide antigen and an antibody against it, for use in the diagnosis, prevention, or treatment of diseases associated with *Pseudomonas aeruginosa.* According to the present invention, there is provided a protein or peptide derived from *Pseudomonas aeruginosa* outer membrane protein PA5158 and an antibody against it, for use in the diagnosis, prevention, or treatment of diseases associated with *Pseudomonas aeruginosa.*

## Description

### Technical Field

The present invention relates to a protein antigen or a peptide antigen derived from a *Pseudomonas aeruginosa* outer membrane protein PA5158, and an antibody against the antigen. The present invention also relates to a vaccine composition comprising the antigen. The present invention further relates to a pharmaceutical composition, a diagnostic agent for a *Pseudomonas aeruginosa* infection, a kit for detecting *Pseudomonas aeruginosa,* and an agent for enhancing *anti-Pseudomonas aeruginosa* activity of an aminoglycoside antibiotic, comprising the antibody.

### Background Art

*Pseudomonas aeruginosa,* which is a gram negative bacillus universally distributed in natural environments such as soil and water, causes serious lethal infections resistant to therapy. Its main targets are compromised patients with attenuated host defense functions generally called compromised hosts including burned, organ-transplanted, and cancer patients. *Pseudomonas aeruginosa* is a main causative bacterium of hospital infections. Furthermore, lung infections caused by this bacterium are lethal to cystic fibrosis patients. An antimicrobial agent having *anti-Pseudomonas aeruginosa* activity is mainly administered to these patients, while many cases do not sufficiently receive a therapeutic effect due to the drug resistance of *Pseudomonas aeruginosa.* Alternatively, vaccines or antibodies against *Pseudomonas aeruginosa* have also been studied for a long time. However, methods directly using inactivated forms of the bacterium had such a disadvantage that various types of vaccines or antibodies must be prepared depending on the different serotypes of *Pseudomonas aeruginosa .*

In such a situation, the prevention or treatment of a *Pseudomonas aeruginosa* infection using passive immunity or active immunity with a *Pseudomonas aeruginosa* protein having a common amino acid sequence among *Pseudomonas aeruginosa* strains has been expected. For example, a recombinant protein in which portions of outer membrane proteins OprF and OprI have been fused with each other (Japanese Patent Laid-Open Publication No. 245699/1996), and a type IV pilin protein (W02004/099250) have been known as applications of such a *Pseudomonas aeruginosa* protein to vaccines.

Moreover, an anti-type IV pilin antibody (W02004/099250), an anti-PA1706 (or PcrV) antibody (U.S. Patent Nos. 6309561 and 6827935), and the like have been reported for antibody drugs targeting a *Pseudomonas aeruginosa* protein.

However, a bacterial protein commonly possessed among clinical isolates of *Pseudomonas aeruginosa,* which exhibit diverse serotypes, can be applied as a "common antigen" to the prevention, diagnosis, or treatment of a *Pseudomonas aeruginosa* infection and as such, has always been demanded.

It has been reported that a PA5158 (also known as OpmG) protein encoded by a PA5158 (or *opmG*) gene is an outer membrane protein that constitutes efflux pumps directly involved in resistance to aminoglycoside antibiotics (Antimicrobial Agents and Chemotherapy, 2003, 47, 1101-1111). However, it has been completely unknown that the protein is used as a vaccine component, and that an antibody against the protein is used as a therapeutic or diagnostic agent for a *Pseudomonas aeruginosa* infection.

### Summary of the Invention

The present inventors have attempted to identify a novel and useful "common antigen" from *Pseudomonas aeruginosa* outer membrane proteins. After various studies, the present inventors have found by GeneChip analysis that a gene encoding a PA5158 (also known as OpmG) protein present in the outer membrane of *Pseudomonas aeruginosa* is constantly expressed regardless of the presence or absence of human sera (Example 1). Moreover, the present inventors have found by gene analysis on 88 clinical isolates of *Pseudomonas aeruginosa* that 2 putative extracellular regions of the PA5158 protein have no detectable amino acid mutation and are completely conserved (Example 2). Furthermore, the present inventors have confirmed that an antiserum or antibody obtained by immunization with a PA5158 recombinant protein exhibits potent protective effect against infections on a *Pseudomonas aeruginosa*-infected model mice (Examples 9 to 12), that the antibody binds to the PA5158 protein (Examples 7, 8, and 13), and that the antibody exhibits an effect of enhancing anti-*Pseudomonas aeruginosa* activity of an aminoglycoside antibiotic (Example 14). The present invention is based on these findings.

An object of the present invention is to provide a protein antigen or a peptide antigen used as a vaccine composition which has an ability to substantially prevent or treat a *Pseudomonas aeruginosa* infection and can respond to the diversity of clinical isolates derived from patients with a *Pseudomonas aeruginosa* infection, or to provide an antibody against the antigen.

According to the present invention, there is provided an antigen composition comprising a protein antigen or a peptide antigen which can induce the production of an antibody against a *Pseudomonas aeruginosa* PA5158 protein.

According to the present invention, there is provided a protein selected from the group consisting of (hereinafter referred to as a "protein according to the present invention"):
(i) a protein comprising the amino acid sequence of SEQ ID NO: 4;
(ii) a protein which comprises an amino acid sequence of SEQ ID NO: 4 in which one or more amino acids are deleted, substituted, inserted, or added, and which is functionally equivalent to a protein consisting of the amino acid sequence of SEQ ID NO: 4;
(iii) a protein which is encoded by a polynucleotide which hybridizes under stringent conditions to a polynucleotide which encodes the amino acid sequence of SEQ ID NO: 4, and which is functionally equivalent to a protein consisting of the amino acid sequence of SEQ ID NO: 4; and
(iv) a protein which comprises an amino acid sequence having 70% or more identity with the amino acid sequence of SEQ ID NO: 4, and which is functionally equivalent to a protein consisting of the amino acid sequence of SEQ ID NO: 4.

According to the present invention, there is provided a peptide comprising the amino acid sequence of SEQ ID NO: 5, or an amino acid sequence of SEQ ID NO: 5 which contains one to several conservative substitutions (hereinafter referred to as a "peptide of a first embodiment according to the present invention").

According to the present invention, there is provided a peptide comprising the amino acid sequence of SEQ ID NO: 6, or an amino acid sequence of SEQ ID NO: 6 which contains one to several conservative substitutions (hereinafter referred to as a "peptide of a second embodiment according to the present invention") (hereinafter, the peptide of the first embodiment according to the present invention and the peptide of the second embodiment according to the present invention are occasionally collectively referred to as "peptide(s) according to the present invention").

According to the present invention, there is provided an antigen composition comprising the protein according to the present invention, or the peptide according to the present invention (hereinafter, this antigen composition, and the antigen composition comprising a protein antigen or a peptide antigen which can induce the production of an antibody against a *Pseudomonas aeruginosa* PA5158 protein are collectively referred to as "antigen composition(s) according to the present invention").

According to the present invention, there is provided a vaccine composition for use in the prevention or treatment of diseases associated with *Pseudomonas aeruginosa,* comprising the antigen composition according to the present invention, and optionally one or more pharmaceutically acceptable carriers, diluents, and/or adjuvants.

According to the present invention, there is provided an antibody against a *Pseudomonas aeruginosa* PA5158 protein or a portion thereof, or a functional fragment thereof (hereinafter referred to as an "antibody according to the present invention").

According to the present invention, there is provided a hybridoma deposited under the accession No. FERM BP-10672.

According to the present invention, there is provided a pharmaceutical composition for use in the prevention or treatment of diseases associated with *Pseudomonas aeruginosa,* comprising the antibody of the first embodiment according to the present invention, and optionally one or more pharmaceutically acceptable carriers and/or diluents.

According to the present invention, there is provided a diagnostic agent for a *Pseudomonas aeruginosa* infection, comprising any of the antibodies of the first to third embodiments according to the present invention.

According to the present invention, there is provided a kit for detecting *Pseudomonas aeruginosa,* comprising any of the antibodies of the first to third embodiments according to the present invention.

According to the present invention, there is provided a potentiator for enhancing anti-*Pseudomonas aeruginosa* activity of an aminoglycoside antibiotic, comprising any of the antibodies of the first to third embodiments according to the present invention.

The present invention provides a vaccine composition and a polyclonal antibody or a monoclonal antibody which have an ability to substantially prevent or treat a *Pseudomonas aeruginosa* infection and further respond to the diversity of clinical isolates derived from patients with a *Pseudomonas aeruginosa* infection. They may be applied to a preventive or therapeutic agent for a *Pseudomonas aeruginosa* infection, or a diagnostic agent for a *Pseudomonas aeruginosa* infection.

Furthermore, the antibody according to the present invention binds to the extracellular regions of the PA5158 protein present in the outer membrane of or extracellularly present in *Pseudomonas aeruginosa.* In addition, it is estimated that these regions are exceedingly highly conservative among strains regardless of serotypes etc., and react with diverse clinical isolates. Therefore, the antibody according to the present invention is expected to have a high therapeutic effect on a *Pseudomonas aeruginosa* infection.

### Brief Description of the Drawing

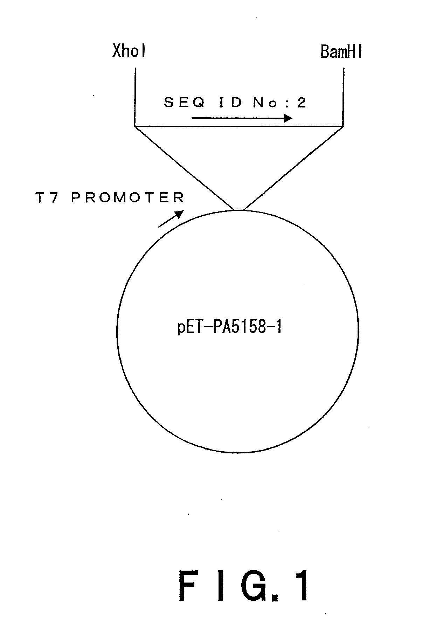

Figure 1 shows a pET15b plasmid (pET-PA5158-1).

### Detailed Description of the Invention

### [PA5158 protein]

A PA5158 protein is an outer membrane PA5158 protein derived from *Pseudomonas aeruginosa.* The amino acid sequence of the protein and the nucleotide sequence of a polynucleotide encoding the protein are described in SEQ ID NO: 3 and SEQ ID NO: 1, respectively.

In this context, based on the signal sequence, and three-dimensional structure information about a *Pseudomonas aeruginosa* PA0427 (OprM) protein (J. Biol. Chem., 2004, 279, 52816-52819) highly homologous to the PA5158 protein, three-dimensional structure information about an *E*. *coli* ToIC protein (Nature, 2000, 405, 914-919), and secondary structure information about the PA5158 protein, it was estimated that a nucleotide sequence of nucleotides 337 to 1479 in 1479 nucleotides as an amino acid coding region of the nucleotide sequence of SEQ ID NO: 1 encodes a cell surface-exposed portion of the PA5158 protein (SEQ ID NO: 2).

The cell surface-exposed portion of the PA5158 protein is a protein selected from the group consisting of:
(i) a protein comprising the amino acid sequence of SEQ ID NO: 4;
(ii) a protein which comprises an amino acid sequence of SEQ ID NO: 4 in which one or more amino acids are deleted, substituted, inserted, or added, and which is functionally equivalent to a protein consisting of the amino acid sequence of SEQ ID NO: 4;
(iii) a protein which is encoded by a polynucleotide which hybridizes under stringent conditions to a polynucleotide which encodes the amino acid sequence of SEQ ID NO: 4, and which is functionally equivalent to a protein consisting of the amino acid sequence of SEQ ID NO: 4; and
(iv) a protein which comprises an amino acid sequence having 70% or more identity with the amino acid sequence of SEQ ID NO: 4, and which is functionally equivalent to a protein consisting of the amino acid sequence of SEQ ID NO: 4.

In the present specification, the expression "one or more amino acids are deleted, substituted, inserted, or added" means that the modification has been carried out according to well-known technical methods such as site-directed mutagenesis, or by substitution of a plurality number of amino acids to an extent of being naturally generated. The number of amino acids to be modified may be preferably 1 to 50, more preferably 1 to 30, further more preferably 1 to 10, still further more preferably 1 to 5, and most preferably 1 or 2.

The modified amino acid sequence can preferably be an amino acid sequence having one or more (preferably, one to several, or 1, 2, 3, or 4) conservative substitutions in the amino acid sequence.

In the present specification, the term "conservative substitution" means that one or more amino acid residues are substituted with other chemically similar amino acid residues. Examples of such conservative substitution include a case where a certain hydrophobic residue is substituted with another hydrophobic residue, and a case where a certain polar residue is substituted with another polar residue having the same electric charge. For every type of amino acids, functionally similar amino acids which can be substituted in such a manner are known in the present technical field. Examples of nonpolar (hydrophobic) amino acids include alanine, valine, isoleucine, leucine, proline, tryptophan, phenylalanine, and methionine. Examples of polar (neutral) amino acids include glycine, serine, threonine, tyrosine, glutamine, asparagine, and cysteine. Examples of positively charged (basic) amino acids include arginine, histidine, and lysine. Examples of negatively charged (acidic) amino acids include aspartic acid and glutamic acid.

In the present specification, the term "under stringent conditions" means conditions wherein a membrane washing procedure after hybridization is carried out at a high temperature in a solution having a low salt concentration. Such washing conditions can be, for example, 0.5×SSC (1×SSC: 15 mM trisodium citrate and 150 mM sodium chloride) at 60°C for 15 minutes, and preferably 0.5×SSC, 0.1% SDS at 60°C for 15 minutes.

Hybridization can be carried out in accordance with a known method. In the case of using a commercially available library, hybridization can be carried out according to the method described in instructions included therewith.

In the present specification, the term "identity" regarding nucleotide sequences or amino acid sequences means the degree of coincidence between the compared sequences in terms of nucleotide residues or amino acid residues that constitute such sequences. The numerical value of such "identity" shown in the present specification may be calculated using a homology search program known to persons skilled in the art. For example, such a numerical value as identity can easily be calculated using a default (initialization) parameter in FASTA or BLAST.

An amino acid sequence having 70% or more identity with the amino acid sequence of SEQ ID NO: 4 can be an amino acid sequence having preferably 80% or more, more preferably 85% or more, further more preferably 90% or more, still further more preferably 95% or more, particularly preferably 98% or more, and most preferably 99% or more identity with the aforementioned amino acid sequence.

In the present invention, if the amino acid sequence of SEQ ID NO: 4 is given, a nucleotide sequence encoding it can easily be determined. Thus, various nucleotide sequences encoding the amino acid sequence of SEQ ID NO: 4 can be selected.

Accordingly, a polynucleotide encoding a protein comprising the amino acid sequence of SEQ ID NO: 4 means not only a part of or the entire DNA sequence of SEQ ID NO: 2 but also a DNA sequence encoding the same amino acids, which has a codon having a degeneracy relationship therewith as a DNA sequence. The present invention further includes an RNA sequence corresponding to such a DNA sequence.

A preferred example of the polynucleotide encoding a protein comprising the amino acid sequence of SEQ ID NO: 4 includes a polynucleotide comprising the nucleotide sequence of SEQ ID NO: 2.

In the present specification, whether or not a certain protein is functionally equivalent to the protein consisting of the amino acid sequence of SEQ ID NO: 4 can be determined by evaluating a biological phenomenon or functions associated with the expression of the protein consisting of the amino acid sequence of SEQ ID NO: 4. For example, it can be determined by allowing the certain protein to express by genetic recombination technique and then evaluating whether or not an antibody against the PA5158 protein can be prepared.

Since the protein according to the present invention is exposed on the cell surface of *Pseudomonas aeruginosa,* the protein can be used as an antigen for preparing an antibody against *Pseudomonas aeruginosa* (protein antigen).

In the cell surface-exposed portion of the PA5158 protein, the following two portions having no detectable amino acid mutation (hereinafter referred to as putative extracellular regions) were identified:
a peptide comprising the amino acid sequence of SEQ ID NO: 5, or an amino acid sequence of SEQ ID NO: 5 which contains one to several conservative substitutions; and
a peptide comprising the amino acid sequence of SEQ ID NO: 6, or an amino acid sequence of SEQ ID NO: 6 which contains one to several conservative substitutions.

Since the peptides according to the present invention are exposed on the cell surface of *Pseudomonas aeruginosa* and conserved in *Pseudomonas aeruginosa,* the peptides can be used as an antigen for preparing an antibody against *Pseudomonas aeruginosa* (peptide antigen).

The peptides were confirmed to have no detectable amino acid mutation in a large number of clinical isolates of *Pseudomonas aeruginosa* and as such, are advantageous in that they are useful as a common antigen.

For the peptides according to the present invention, a blocking group may be added to the N- or C-terminals thereof, for example, so as to prevent aggregation attributed to electric charges. Acetylation and amidation were often used for the N- and C-terminals, respectively, but not limited to these. The peptides according to the present invention can be modified, for example, by adding a cysteine residue thereto, so as to enhance binding with a spacer.

### Sulfo-SMCC

(Sulfosuccinimidyl-4-[N-maleimidomethyl]cyclohexane-1-carbox ylate) or the like is generally used as the spacer, but not limited to this. A compound functioning as a spacer can be used.

For the peptides according to the present invention, carrier proteins such as bovine serum albumin (BSA), ovalbumin (OVA), human serum albumin (HSA), or keyhole limpet hemocyanin (KLH) can be used as carriers, but not limited to these.

### [Antigen composition]

The protein according to the present invention or the peptides according to the present invention can be used as a protein antigen or a peptide antigen. Thus, according to the present invention, there is provided an antigen composition comprising the protein antigen or the peptide antigen which can induce the production of an antibody against a *Pseudomonas aeruginosa* outer membrane PA5158 protein.

In this context, the protein antigen or the peptide antigen can preferably be used by purifying the protein according to the present invention or the peptides according to the present invention according to a method well known to persons skilled in the art.

In the present specification, the term "antigen composition" may be a composition consisting of only the protein antigen, or the peptide antigen or a composition comprising such an antigen and other components.

According to the present invention, there is provided an antigen composition comprising a protein antigen or a peptide antigen which can induce the production of an antibody against a *Pseudomonas aeruginosa* PA5158 protein.

### [Vaccine composition]

The antigen compositions according to the present invention can be used as a vaccine. Thus, according to the present invention, there is provided a vaccine composition comprising an antigen composition which can induce the production of an antibody against a *Pseudomonas aeruginosa* outer membrane PA5158 protein.

According to the present invention, a vaccine composition for use in the prevention or treatment of diseases associated with *Pseudomonas aeruginosa,* comprising the antigen composition according to the present invention, and optionally one or more pharmaceutically acceptable carriers, diluents, and/or adjuvants can be prepared.

The carriers used in the vaccine composition according to the present invention are selected based on the mode and route of administration, and actual standard drug formulation and may be, for example, carrier proteins (e.g., bovine serum albumin (BSA), ovalbumin (OVA), human serum albumin (HSA), and keyhole limpet hemocyanin (KLH)), solubilizers (e.g., ethanol, polysorbate, and Cremophor EL^{™}), isotonic agents, preservatives, antioxidants, excipients (e.g., lactose, starch, crystalline cellulose, mannitol, maltose, calcium hydrogen phosphate, light anhydrous silicic acid, and calcium carbonate), binders (e.g., starch, polyvinylpyrrolidone, hydroxypropylcellulose, ethylcellulose, carboxymethylcellulose, and gum arabic), lubricants (e.g., magnesium stearate, talc, and hydrogenated oil), and stabilizers (e.g., lactose, mannitol, maltose, polysorbate, macrogol, and polyoxyethylene hydrogenated castor oil). Glycerin, dimethylacetamide, 70% sodium lactate, a surfactant, or a basic substance (e.g., sodium hydroxide, ethylenediamine, ethanolamine, sodium bicarbonate, arginine, meglumine, or trisaminomethane), etc. may be added, if necessary.

Specifically, the peptides according to the present invention can be coupled to a known KLH solution (Calbiotec, 125 mg is dissolved per ml of a 50% glycerol solution) as a carrier protein, so as to enhance the antigenicity of the vaccine composition according to the present invention.

The diluents used in the vaccine composition according to the present invention are selected based on the mode and route of administration, and actual standard drug formulation. Examples of the diluents include water, a saline, a phosphate-buffered saline, and a bicarbonate solution.

The adjuvants used in the vaccine composition according to the present invention are selected based on the mode and route of administration, and actual standard drug formulation. Examples of the adjuvants include cholera toxin, *E. coli* heat-labile enterotoxin (LT), liposome, and an immunostimulating complex (ISCOM).

An administration route may differ depending on the age, body weight, sex, and general health of a recipient at risk of a *Pseudomonas aeruginosa* infection, but adminiatration can be carried out by any of oral administration and parenteral administration (e.g., intravenous injection, intraarterial injection, and local administration) routes. Among them, parenteral administration is preferable.

The dosage form for oral administration and parenteral administration and the preparation method thereof are well known to persons skilled in the art. The dosage form for oral administration and parenteral administration can be prepared by a conventional process, for example, by mixing the antigen compositions according to the present invention, for example, with the aforementioned pharmaceutically acceptable carriers.

Examples of a dosage form for oral administration include solid and liquid dosage forms such as a solution, a tablet, a granule, a powder, or a capsule.

Examples of a dosage form for parenteral administration include a solution, a suspension, an ointment, a cream, a suppository, an ophthalmic agent, nasal drops, and ear drops.

If the sustained release of the present preparation is desired, a biodegradable polymer (e.g., poly-D,L-lactide-co-glycoside or polyglycoside) can be added as a bulk matrix (see e.g., U.S. Patent Nos. 5,417,986, 4,675,381, and 4,450,150).

In the case of oral administration, a flavoring agent and a coloring agent can also be added.

Appropriate pharmaceutical carriers and diluents and the like, and pharmaceutical necessities for use thereof are described in Remington's Pharmaceutical Sciences.

A dose of the vaccine composition according to the present invention is determined by the present inventor based on the type of a vaccine antigen, the possibility of administration of the present antigen in combination with adjuvants, the type of the adjuvants coadministered therewith, the mode and frequency of administration, and a desired effect (e.g., a preventive or therapeutic effect), and may be generally 1 µg/dose to 100 mg/dose per adult. When the present vaccine is administered with adjuvants, the dose may be generally 1 ng/dose to 1 mg/dose per adult. Such a dose may be administered several times according to decision of the present inventor, as necessary. For example, initial vaccination and subsequent 3 booster vaccinations at 1-week intervals can be carried out. Alternatively, a booster injection and a second booster injection can be carried out on the 8th to 12th week and 16th to 20th week, respectively, from the first immunization, using the same formulations.

### [Antibody]

The antibody according to the present invention can recognize a *Pseudomonas aeruginosa* outer membrane PA5158 protein or a portion thereof, and bind to the *Pseudomonas aeruginosa .*

According to the present invention, there is provided an antibody according to the present invention or a functional fragment thereof, wherein the portion of the *Pseudomonas aeruginosa* PA5158 protein is a cell surface-exposed portion of the *Pseudomonas aeruginosa* PA5158 protein.

According to the present invention, there is provided an antibody according to the present invention, wherein the portion of the *Pseudomonas aeruginosa* PA5158 protein is the protein according to the present invention (hereinafter referred to as an "antibody of a first embodiment according to the present invention").

According to the present invention, there is provided an antibody according to the present invention, wherein the portion of the *Pseudomonas aeruginosa* PA5158 protein is the peptide of the first embodiment according to the present invention (hereinafter referred to as an "antibody of a second embodiment according to the present invention").

According to the present invention, there is provided an antibody according to the present invention, wherein the portion of the *Pseudomonas aeruginosa* PA5158 protein is the peptide of the second embodiment according to the present invention (hereinafter referred to as an "antibody of a third embodiment according to the present invention").

Such an antibody includes an antibody that recognizes the protein according to the present invention and binds to the *Pseudomonas aeruginosa.* It also includes an antibody that recognizes the peptide according to the present invention.

The antibody according to the present invention is preferably obtained by immunizing an experimental animal with an antigen composition comprising the purified protein antigen or peptide antigen according to the present invention, which is administered in an amount that can induce the antibody. Such an antibody can be used as a pure antibody by collecting blood from the heart or artery, separating antisera therefrom, and purifying the obtained antisera.

The antibody according to the present invention includes: a polyclonal antibody or monoclonal antibody obtained by using the PA5158 protein or peptide as an antigen and immunizing a mammal such as a mouse with the aforementioned antigen (which includes a monoclonal antibody produced by a hybridoma that produces the monoclonal antibody of the present invention); a chimeric antibody and a humanized antibody prepared by genetic recombination technique; and a human antibody prepared using a human antibody-producing transgenic animal or the like.

When the antibody according to the present invention is administered as a medicament to a human, a human antibody is preferably used in terms of reducing side effects.

The "human antibody" means an antibody wherein all regions are derived from humans. The human antibody according to the present invention can be prepared using a method well known to persons skilled in the art (see e.g., Intern. Rev. Immunol, 1995, 13, 65-93; J. Mol. Biol, 1991, 222, 581-597; Japanese Patent Laid-Open Publication No. 146194/1998; Japanese Patent Laid-Open Publication No. 155492/1998; Japanese Patent No. 2938569; Japanese Patent Laid-Open Publication No. 206387/1999; Japanese Patent Laid-Open Publication No. 509612/1996; and Japanese Patent Laid-Open Publication No. 505107/1999).

The "humanized antibody" is an antibody prepared by transplanting only the gene sequence of the antigen-binding site (CDR; complementarity determining region) of a mouse antibody into a human antibody gene (CDR grafting). The humanized antibody according to the present invention can be prepared using a method well known to persons skilled in the art (see e.g., EP239400 and WO90/07861).

The "chimeric antibody" is an antibody prepared by ligating the variable region of a certain antibody to the constant region of an antibody of different species therefrom. Specifically, a mouse is immunized with an antigen, and an antibody variable region (V region) that binds to the antigen is cut out of the gene of the mouse monoclonal antibody. The thus obtained V region is then allowed to bind to an antibody constant region (C region) gene derived from human bone marrow, so as to prepare a chimeric antibody. The chimeric antibody according to the present invention can be prepared using a method well known to persons skilled in the art (see e.g., Japanese Patent Laid-Open Publication No: 280387/1996 and U.S. Patent Nos. 4816397, 4816567, and 5807715).

The monoclonal antibody according to the present invention can be prepared using a method well known to persons skilled in the art (see e.g., Antibodies: A Laboratory Manual, Ed Harlow and David Lane, Cold Spring Harbor Laboratory (1988); Experimental Manual for Monoclonal Antibody, edited by Sakuji Toyama et al., Kodansha, (1987); and Monoclonal Antibody: Hybridoma and ELISA, edited by Tatsuo Iwasaki, et al., Kodansha (1987)).

The polyclonal antibody according to the present invention can be prepared using a method well known to persons skilled in the art.

The "functional fragment" according to the present invention means a part of an antibody (a partial fragment), which specifically recognizes the protein according to the present invention. Specific examples of such a functional fragment include Fab, Fab', F(ab')₂, a variable region fragment (Fv), disulfide-bonded Fv, and a single-chain antibody (scFv), and a polymer thereof.

Preferred examples of the antibody of the first embodiment according to the present invention include an antibody against a protein comprising the amino acid sequence of SEQ ID NO: 4, or an amino acid sequence of SEQ ID NO: 4 which contains one to several conservative substitutions, and a functional fragment thereof.

Preferred examples of the antibody of the second embodiment according to the present invention include a monoclonal antibody produced by a hybridoma deposited under FERM BP-BP-10672.

Accordingly, according to the present invention, there is provided a hybridoma (5158-L1-1) deposited with the National Institute of Advanced Industrial Science and Technology, International Patent Organism Depositary (AIST Tsukuba Central 6, Higashi 1-1-1, Tsukuba, Ibaraki, 305-8566, Japan), under the accession No. FERM BP-10672 (transferred from FERM P-20675) on October 7, 2005.

The antibody according to the present invention is preferably a monoclonal antibody.

According to the present invention, there is provided a monoclonal antibody cross-reactive with the same antigen as that of the monoclonal antibody produced by the hybridoma according to the present invention.

According to the present invention, there is provided an antibody that can bind to *Pseudomonas aeruginosa,* which is produced by the animal's own immune system in response to the antigen compositions according to the present invention.

### [Use of antibody and pharmaceutical composition]

### Diseases associated with Pseudomonas aeruginosa

*Pseudomonas aeruginosa* is a pathogen of opportunistic infections which cause lethal consequences with reductions in the resistance of hosts. Moreover, *Pseudomonas aeruginosa* is resistant to antibiotics and is therefore a main causative bacterium of hospital infections. As shown later in examples, it was confirmed that the antibody of the first embodiment according to the present invention actually has a protective effect against infections on a *Pseudomonas aeruginosa*-susceptible murine model with macrophage functions reduced by administration of mucin, and a *Pseudomonas* aeruginosa-susceptible murine model with a neutrophil level reduced by administration of Cyclophosphamide monohydrate (Examples 9 and 10). It was also confirmed that the antibody of the first embodiment according to the present invention actually has protective effect against infections on a multidrug resistant *Pseudomonas* aeruginosa-susceptible murine model (Examples 11 and 12). Thus, the antibody of the first embodiment according to the present invention is useful for the prevention or treatment of diseases associated with *Pseudomonas aeruginosa.*

Examples of the diseases associated with *Pseudomonas aeruginosa* include systemic infectious diseases caused by a *Pseudomonas aeruginosa* infection including a multidrug resistant *Pseudomonas aeruginosa* infection, for example, septicemia, meningitis, and endocarditis. Alternative examples of the diseases associated with *Pseudomonas aeruginosa* include: otitis media and sinusitis in the otolaryngologic field; pneumonia, chronic respiratory tract infection, and catheter infection in the pulmonological field; postoperative peritonitis and postoperative infection in a biliary duct or the like in the surgical field; abscess of eyelid, dacryocystitis, conjunctivitis, corneal ulcer, corneal abscess, panophthalmitis, and orbital infection in the ophthalmological field; and urinary tract infections (including complicated urinary tract infection), catheter infection, and abscess around the anus in the urologic field. Further examples thereof include burns (including a serious burn and a burn of the respiratory tract), decubital infection, and cystic fibrosis.

The antibody of the first embodiment according to the present invention is useful in that it is expected to be effective for the prevention or treatment of a multidrug resistant *Pseudomonas aeruginosa* infection which is difficult to treat.

According to the present invention, there is provided use of the antibody of the first embodiment according to the present invention for producing a preventive agent or therapeutic agent for diseases associated with *Pseudomonas aeruginosa.*

According to the present invention, there is provided a method for preventing or treating diseases associated with *Pseudomonas aeruginosa,* comprising the step of administering a preventively or therapeutically effective amount of the antibody of the first embodiment according to the present invention to mammals including a human.

### Diagnostic agent for Pseudomonas aeruginosa infection

As shown later in examples, it was confirmed that the antibody of the first embodiment according to the present invention binds to each of the protein according to the present invention, the peptide of the first embodiment according to the present invention, and the peptide of the second embodiment according to the present invention, and reacts with only *Pseudomonas aeruginosa* and with no other bacterial species (Examples 7 and 8). It was also confirmed that the antibody of the second embodiment according to the present invention binds to the peptide of the first embodiment according to the present invention (Example 13). It was further confirmed that the antibody of the third embodiment according to the present invention binds to the peptide of the second embodiment according to the present invention, and reacts with only *Pseudomonas aeruginosa* and with no other bacterial species (Example 7). Thus, the antibody according to the present invention is useful for the diagnosis of a *Pseudomonas aeruginosa* infection.

The antibody according to the present invention can be used not only as a purified product but also in the form of a hybridoma culture supernatant or ascites, so as to differentiate a *Pseudomonas aeruginosa* infection from other bacterial species infections.

According to the present invention, there is provided a method for diagnosing a *Pseudomonas aeruginosa* infection using the antibody according to the present invention.

The diagnosis method according to the present invention can be carried out by collecting biological samples such as sputum, a lung lavage fluid, pus, tears, blood, or urine from mammals including a human at risk of a *Pseudomonas aeruginosa* infection, subsequently contacting the collected samples with the antibody according to the present invention, and determining whether or not an antigen-antibody reaction occurs.

### Diagnostic agent kit for Pseudomonas aeruginosa infection

According to the present invention, there is provided a kit for detecting the presence of *Pseudomonas aeruginosa,* comprising at least the antibody according to the present invention.

The antibody according to the present invention may be labeled. This detection kit detects the presence of *Pseudomonas aeruginosa* by detecting an antigen-antibody reaction.

Thus, the detection kit according to the present invention can further contain various types of reagents for carrying out an antigen-antibody reaction, a secondary antibody used, for example, in ELISA, a chromogenic reagent, a buffer, instructions, and/or an instrument, etc., if desired.

### Potentiator for enhancing anti-Pseudomonas aeruginosa activity of an aminoglycoside antibiotic

As shown later in examples, it was confirmed that addition of the antibody according to the present invention enhances antimicrobial activity of an aminoglycoside antibiotic gentamicin (Example 14). Thus, the antibody according to the present invention can be used as a potentiator for enhancing anti*-Pseudomonas aeruginosa* activity.

According to the present invention, there is provided use of the antibody according to the present invention for producing a potentiator for enhancing *anti-Pseudomonas aeruginosa* activity of an aminoglycoside antibiotic.

According to the present invention, there is provided a method for enhancing anti*-Pseudomonas aeruginosa* activity of an aminoglycoside antibiotic, comprising the step of administering a therapeutically effective amount of the antibody according to the present invention to mammals including a human.

### Pharmaceutical composition

The pharmaceutical composition or agent according to the present invention may be used in the form of a composition comprising the antibody according to the present invention as an active ingredient and preferably containing the purified antibody, and optionally other components, for example, a saline, an aqueous glucose solution, and a phosphate buffer.

The pharmaceutical composition according to the present invention may be formulated as a liquid or freeze-dried form, as necessary. Such a pharmaceutical composition may optionally contain pharmaceutically acceptable carriers, for example, a stabilizer, a preservative, and an isotonic agent.

Examples of the pharmaceutically acceptable carriers can include: mannitol, lactose, saccharose, and human albumin for a freeze-dried preparation; and a saline, water for injections, a phosphate buffer, and aluminum hydroxide for a liquid preparation, but not limited to these.

An administration route may differ depending on the age, body weight, sex, and general health of a recipient, but administration can be carried out by any of oral administration and parenteral administration (e.g., intravenous injection, intraarterial injection, and local administration) routes. Among them, parenteral administration is preferable.

A dose of the pharmaceutical composition differs depending on the age, body weight, sex, and general health of a patient, the severity of a *Pseudomonas aeruginosa* infection, and components of an antibody composition to be administered. A daily dose of the antibody composition according to the present invention is generally 0.1 to 1000 mg/kg of body weight, preferably 1 to 100 mg/kg of body weight, per adult for intravenous injection.
It is preferable that the pharmaceutical composition according to the present invention should be administered in advance to a patient at risk of a *Pseudomonas aeruginosa* infection.

When the pharmaceutical composition is prepared as a diagnostic agent, this diagnostic agent can be obtained in any dosage form by adopting any means suitable for the purpose. For example, ascites, a culture solution containing the antibody of interest, or the purified antibody is measured for its antibody titer and appropriately diluted with PBS (phosphate buffer containing a saline) or the like, and a preservative such as 0.1% sodium azide is then added thereto. Alternatively, the antibody of the present invention adsorbed on latex or the like is also determined for its antibody titer and appropriately diluted, and a preservative is added thereto for use. Such an antibody of the present invention bound with latex particles is one of preferable dosage forms as a diagnostic agent. In this case, appropriate resin materials, for example, polystyrene, polyvinyl toluene, or polybutadiene, are suitable as the latex.

### Examples

Hereinafter, the present invention will be described with reference to examples for promoting understanding of the present invention. However, the present invention is not intended to be limited to these examples.

### Example 1: GeneChip analysis

GeneChip expression analysis system (Affymetrix, GeneChip *P. aeruginosa* genome array) was used as an approach for identifying genes that are expressed in a medium supplemented with human sera. Shake culture was carried out using *Pseudomonas aeruginosa* PA01 strains (ATCC BAA-47) under 3 different culture conditions, that is, in Luria-Bertani (LB) media (Nacalai Tesque) supplemented with 0%, 20%, and 50% human sera (the final composition of LB media was equal among them) at 37°C until absorbance at 595 nm reached 1.0. Using RNeasy Protect Bacteria Mini kit (QIAGEN GmbH), total RNA was extracted according to the method described in documents included therewith and quantified using 2100 bioanalyzer (Agilent Technologies). Then, experiments were carried out according to the method described in documents included with GeneChip. Gene expression data was analyzed using Microarray Suite 5.0 (Affymetrix), and signal and detection were calculated. At this time, correction was carried out, such that the average value of signals from all probe sets was 1000. Two independent experiments were carried out.

As a result, a PA4761 protein (DnaK or HSP70), which is a house keeping protein, was determined, under all the culture conditions regardless of the presence or absence of added sera, to be "Present" that indicates that a transcription product was detected, and it was thus shown that the gene is expressed. Moreover, a PA2018 protein (MexY) (J. Bacteriology, 2005, 187, 5341-5346), which is an inner membrane-spanning protein that associates with PA5158 and PA2019 (MexX) proteins so as to constitute drug efflux pumps and is induced by ribosome inhibitors such as tetracycline or aminoglycoside antibiotics, was determined, under these conditions free of these drugs, to be "Absent", and it was thus shown that the gene is not expressed. By contrast, a PA5158 protein was determined to be "Present", under all the conditions regardless of the presence or absence of added sera.

Thus, the PA5158 gene is certainly expressed, and the possibility that its gene product PA5158 protein is constantly present on the bacterial surface was suggested. This suggested that the *Pseudomonas aeruginosa* PA5158 protein is useful as a vaccine component.

### Example 2: Analysis of PA5158 gene in clinical isolates

Bacterial strains used were 88 *Pseudomonas aeruginosa* strains (stored in Yokohama Research Lab., Meiji Seika Kaisha) isolated from various types of clinical materials in clinical facilities across Japan, and were subjected to tests. These strains were derived from blood, urine, sputum, pus, pharyngeal mucus, and the like, and their serotypes include group A, B, E, G, I, etc., based on serological classification according to the decision of the serotyping committee sponsored by Japan Pseudomonas aeruginosa Society (1975).

### (1) Preparation of genomic DNA

Each of 88 clinical isolates of *Pseudomonas aeruginosa* was cultured overnight at 37°C in a Mueller-Hinton medium (Becton Dickinson) and collected by low-speed centrifugation. Using DNeasy Tissue kit (QIAGEN GmbH), genomic DNA was prepared from the obtained bacterial cells according to the method described in documents included therewith.

### (2) Amplification of DNA fragment by the PCR method

Using the prepared genomic DNA as a template, a region containing the PA5158 gene was amplified by PCR. Specifically, a primer set (SEQ ID NO: 7 and SEQ ID NO: 8) for specifically amplifying each gene was designed based on the *Pseudomonas aeruginosa* PAO1 genomic sequence (NCBI accession No. NC_002516). Using GeneAmp PCR System 9700 (Applied Biosystems), PCR was carried out using Takara ExTaq (Takara Bio) according to instructions included therewith. The DNA fragment thus amplified by PCR was confirmed by agarose gel electrophoresis to have the size of interest (1645 bp).

### (3) Analysis of polynucleotide sequence using DNA sequencer

The PCR product was purified using MultiScreen PCR plate (Millipore Corporation) and then subjected to a sequencing reaction. Primers (SEQ ID NO: 9 to SEQ ID NO: 12) capable of sequencing each PCR product were designed based on the PAO1 genomic sequence (NC_002516). BigDye Terminator v1.1 Cycle Sequencing kit (Applied Biosystems) was used in a sequencing reaction. The sequencing reaction was carried out using GeneAmp PCR System 9700 (Applied Biosystems) according to instructions included therewith. The sequencing reaction product was purified using MultiScreen-HV plate (Millipore Corporation) filled with Sephadex G-50 Fine DNA Grade (Amersham Biosciences AB) swollen with water in advance. Then, the polynucleotide sequence was analyzed using Applied Biosystems 3730 DNA Analyzer (Applied Biosystems).

The polynucleotide sequences in the clinical isolates determined by the analysis were converted to polypeptide sequences, and these polypeptide sequences were compared with those from the PAO1 strain. As a result, 21 mutations were observed in the full-length sequence of the PA5158 protein (Table 1).

However, 2 putative extracellular regions (SEQ ID NO: 5 and SEQ ID NO: 6) predicted by the present inventors based on three-dimensional structure information about a *Pseudomonas aeruginosa* PA0427 (OprM) protein (J. Biol. Chem., 2004, 279, 52816-52819) highly homologous to the PA5158 protein, three-dimensional structure information about an *E*. *coli* ToIC protein (Nature, 2000, 405, 914-919), and secondary structure information about the PA5158 protein, were confirmed to have no detectable amino acid mutation. This suggested that the *Pseudomonas aeruginosa* PA5158 protein is useful as a "common antigen."

**[Table 1]**

| Mutation pattern of the PA5158 protein in clinical isolates | | | | | | | | | | | | | | | | | | | | | | |
|---|---|---|---|---|---|---|---|---|---|---|---|---|---|---|---|---|---|---|---|---|---|---|
| | Amino acid No. | | | | | | | | | | | | | | | | | | | | | |
| Pattern | 3 | 5 | 8 | 14 | 20 | 36 | 37 | 50 | 53 | 67 | 117 | 171 | 215 | 279 | 388 | 397 | 405 | 409 | 416 | 499 | 477 | The Number of strains |
| PA 01 type | F | L | P | A | L | R | L | D | A | G | S | R | L | N | V | D | E | I | D | A | Q | 9 |
| 1 | - | P | L | - | - | - | - | - | - | - | - | - | - | - | - | N | - | - | - | T | - | 12 |
| 2 | - | - | - | - | v | - | - | - | - | - | - | - | - | - | - | N | - | - | - | - | - | 11 |
| 3 | - | - | - | - | - | Q | - | - | - | - | - | - | - | - | - | - | - | - | - | - | - | 10 |
| 4 | - | P | - | - | - | - | - | - | - | - | - | - | - | - | - | - | A | V | - | - | - | 2 |
| 5 | - | P | L | V | - | - | - | - | - | - | - | - | - | - | - | - | - | - | - | - | - | 14 |
| 6 | - | - | - | - | - | - | - | - | - | - | - | - | - | - | - | N | - | - | - | - | - | 3 |
| 7 | - | P | L | - | - | - | - | E | - | - | - | - | - | - | - | - | - | - | - | - | - | 1 |
| 8 | - | P | L | - | - | - | - | E | - | - | - | - | - | - | - | - | - | - | - | - | - | 2 |
| 9 | - | - | - | - | - | - | - | - | - | - | - | - | - | - | A | - | - | - | - | - | - | 1 |
| 10 | - | P | L | - | - | - | P | E | - | - | - | - | - | - | - | N | - | - | - | - | - | 1 |
| 11 | - | P | L | - | - | - | - | E | - | - | - | - | - | - | - | N | - | - | - | - | - | 1 |
| 12 | - | P | L | V | - | - | - | - | - | - | - | - | - | - | - | - | A | V | - | - | - | 2 |
| 13 | - | - | - | - | - | Q | - | - | - | - | - | - | M | - | - | - | - | - | - | - | - | 1 |
| 14 | - | - | - | - | - | Q | - | - | - | - | - | - | - | - | - | - | A | V | - | - | - | 1 |
| 15 | - | P | L | V | - | - | - | - | - | - | - | - | - | - | - | - | - | - | N | - | - | 2 |
| 16 | - | - | - | - | - | Q | - | - | - | - | - | - | - | - | - | N | - | - | - | T | - | 1 |
| 17 | - | p | - | - | - | - | P | E | - | - | - | - | - | D | - | - | - | - | - | - | - | 1 |
| 18 | - | - | - | V | - | - | - | - | - | - | - | - | - | - | - | - | - | - | N | - | - | 1 |
| 19 | - | p | L | V | - | - | - | - | - | - | - | - | - | - | - | N | - | - | - | - | - | 1 |
| 20 | - | - | - | - | - | - | - | - | - | R | - | - | - | - | - | N | - | - | - | - | - | 2 |
| 21 | - | p | L | - | - | - | P | - | - | - | - | - | - | - | - | - | - | - | - | - | - | 1 |
| 22 | - | - | - | - | - | - | - | - | - | - | - | - | - | - | - | N | K | - | - | - | - | 2 |
| 23 | - | - | - | - | - | Q | - | - | - | - | - | L | - | - | - | - | - | V | - | - | H | 1 |
| 24 | - | p | L | V | - | - | - | - | - | - | - | - | - | - | - | - | - | V | - | - | - | 1 |
| 25 | - | p | - | - | - | - | - | - | - | - | - | - | - | - | - | N | - | - | - | T | - | 1 |
| 26 | L | - | - | - | - | - | - | - | V | - | - | - | - | - | - | - | - | - | - | - | - | 1 |
| 27 | - | p | - | V | - | - | - | - | - | - | - | - | - | - | - | - | - | - | - | - | - | 1 |
| 28 | - | - | - | - | - | Q | - | - | - | - | - | - | - | - | - | - | A | - | - | - | - | 1 |
| Total | | | | | | | | | | | | | | | | | | | | | | 88 |

| | | | | | | | | | | | | | | | | | | | | | | |
|---|---|---|---|---|---|---|---|---|---|---|---|---|---|---|---|---|---|---|---|---|---|---|
| "-" represents the same amino acid as that in the PA01 strain. | | | | | | | | | | | | | | | | | | | | | | |

### Example 3: Cloning of the PA5158 gene DNA fragment

A DNA fragment (SEQ ID NO: 2) from nucleotides 337 to 1479 in 1479 nucleotides as an amino acid coding region of the *Pseudomonas aeruginosa* PA5158 gene (SEQ ID NO: 1) was incorporated into a cell-free protein expression vector pIVEX2.4d (Roche Diagnostics) and an *E. coli* expression vector pET15b (Novagen) by the following method.

Based on the signal sequence, and three-dimensional structure information about a *Pseudomonas aeruginosa* PA0427 (OprM) protein (J. Biol. Chem., 2004, 279, 52816-52819) highly homologous to the PA5158 protein, three-dimensional structure information about an *E*. *coli* ToIC protein (Nature, 2000, 405, 914-919), and secondary structure information about the PA5158 protein, it was estimated that a nucleotide sequence of nucleotides 1 to 336 in the amino acid coding region encodes a cell surface-unexposed region. Thus, this nucleotide sequence was excluded from cloning.

The DNA fragment to be cloned was amplified from the *Pseudomonas aeruginosa* PAO1 genomic DNA by PCR (GeneAmp PCR System 9600; Perkin-Elmer). Pyrobest (Takara Bio) was used as DNA polymerase. A reaction solution was supplemented with 10% dimethyl sulfoxide. Primers (SEQ ID NO: 13 and SEQ ID NO: 14) containing nucleotides for adding restriction sites XhoI (CTCGAG) and BamHI (GGATCC) were used as PCR primers.

Temperature conditions involved heating at 94°C for 2 minutes, subsequent 30 cycles consisting of 94°C-30 seconds, 54°C-1 minute, and 72°C-2 minutes, and final reaction at 72°C for additional 5 minutes. The PCR product was purified using QIAquick PCR Purification Kit (Qiagen), and the purified product was digested with XhoI (New England Biolabs) and BamHI (Toyobo). pIVEX2.4d was digested with XhoI and BamHI. These DNA fragments were electrophoresed on agarose gel, and extracted and purified using QIAquick Gel Extraction Kit (Qiagen). The PCR product and pIVEX2.4d thus digested with XhoI-BamHI were ligated using T4 DNA ligase (Ligation High, Toyobo), and *E*. *coli* DH5α strains (Competent High DH5α, Toyobo) were transformed with the ligation product. The pIVEX2.4d plasmid in which the PA5158 gene fragment had been incorporated (pIVEX-PA5158-10) was purified using QIAprep Spin Miniprep Kit (Qiagen), and the nucleotide sequence of the insert was confirmed (BigDye Terminator v1.1 Cycle Sequencing Kit, Applied Biosystems).

Next, pET15b was digested with XhoI and BamHI, and ligated to the XhoI-BamHI insert fragment of pIVEX-PA5158-10. *E. coli was* transformed with the ligation product, so as to obtain a pET15b plasmid in which the PA5158 gene fragment had been incorporated (pET-PA5158-1) (Figure 1).

### Example 4: Expression and purification of the PA5158 recombinant protein

Cell-free and E. *coli* expression systems were used in expression of a recombinant protein.

RTS 500 ProteoMaster E. coli HY Kit (Roche Diagnostic) for carrying out transcription and translation using T7 RNA polymerase and E. *coli* lysates was used in a cell-free system. The cell-free protein expression vector pIVEX-PA5158-10 is a plasmid that encodes the PA5158 protein, in which His-tag (6 consecutive histidines) has been fused downstream of a T7 promoter (see Example 3). A cell-free reaction solution was prepared according to instructions. A reaction was carried out at 30°C for 20 hours by addition of 10 µg of pIVEX-PA5158-10, and the produced insoluble protein was collected by centrifugation.

*E. coli* BL21 (DE3) strains in which a T7 RNA polymerase gene has been incorporated, and a pET vector expression system (Novagen) having a T7 promoter were used in an E. *coli* expression system. In this expression system, transcription of the T7 RNA polymerase gene incorporated in the chromosome of the BL21 (DE3) strain is suppressed by a lacI repressor, and this suppression of transcription is removed by addition of an inducer isopropyl-β-D-thiogalactoside (IPTG), so as to induce expression of T7 RNA polymerase. Moreover, transcription of the gene incorporated downstream of the T7 promoter of the pET vector is also suppressed by a lacI repressor, and this suppression of transcription is removed by addition of IPTG, so as to induce expression of the incorporated gene through transcription caused by the T7 RNA polymerase supplied from the host. The *E. coli* expression vector pET-PA5158-1 is a plasmid that encodes the PA5158 protein, in which a histidine tag has been fused downstream of a T7 promoter (see Example 3). The BL21 (DE3) strains were treated with calcium chloride (see Molecular Cloning 2nd ed., Sambrook et al. (1989)) and transformed with pET-PA5158-1. The transformants were cultured overnight in an LB medium containing 50 µg/ml ampicillin, and they were suspended (200 fold diluted) in a fresh medium and cultured at 37°C for 4 hours. IPTG was then added at a final concentration of 0.5 mM, and culture was continued for additional 3 hours. The cells were collected by centrifugation and frozen at -20°C. The cells were dissolved in BugBuster Protein Extraction Reagent (Novagen), and inclusion bodies were collected according to instructions included therewith. In this procedure, ultrasonication was also carried out, and lysozyme (egg-white lysozyme, Seikagaku Corporation) was used at a final concentration of 200 µg/ml.

Ni chelate chromatography using the His-tag was used in protein purification. The insoluble protein expressed in the cell-free or *E*. *coli* expression system was solubilized with a dissolution buffer (Dulbecco's phosphate-buffered saline (PBS) supplemented with 8 M urea, 5 mM imidazole, 200 mM NaCl, and 0.1% NP-40). The dissolved protein was bound to Ni-NTA Agarose (Qiagen) and washed with 40 volumes of a dissolution buffer. The protein was further washed with 40 volumes of a wash buffer (having the same composition as that of the dissolution buffer except for NP-40). Then, the His-tag-fused protein was eluted with an elution buffer (PBS supplemented with 8 M urea, 300 mM imidazole, and 200 mM NaCl), followed by collection.

As a result, 1.5 mg of the protein and 9.0 mg of the protein were finally obtained from 1 ml of the reaction solution in the cell-free system and 100 ml of the culture in the E. *coli* expression system, respectively.

### Example 5: Immunization with antigen and preparation of sera

*Pseudomonas aeruginosa* PA103 strains (ATCC29260) were cultured overnight at 37°C on a Mueller-Hinton agar medium. A few colonies were suspended in an LB medium and then shake-cultured overnight at 37°C, and they were washed with PBS, followed by resuspension. Then, inactivation treatment was carried out for 24 hours or longer by addition of 1% formalin, and the strains thus inactivated were used.

For use in immunization, the PA5158 recombinant protein was dissolved in an 8 M urea solution, resulting in a concentration of 100 µg/ml.

The amino acid sequences of the extracellular regions in the PA5158 protein were estimated by the present inventors based on three-dimensional structure information about a *Pseudomonas aeruginosa* PA0427 (OprM) protein (J. Biol. Chem., 2004, 279, 52816-52819) highly homologous to the PA5158 protein, three-dimensional structure information about an *E*. *coli* ToIC protein (Nature, 2000, 405, 914-919), and secondary structure information about the PA5158 protein. Peptides containing the estimated amino acid sequences (SEQ ID NO: 5 and SEQ ID NO: 6) were synthesized by a solid-phase synthesis method using Fmoc. A cysteine residue was added to the amino terminal of each amino acid sequence of SEQ ID NO: 5 and SEQ ID NO: 6. Peptides having an acetylated amino terminal and an amidated carboxyl terminal were synthesized. [M+1] m/z 1664.2 (calculated value: m/z 1663.9) was observed by mass spectrometry in the synthetic peptide containing the amino acid sequence of SEQ ID NO: 5 (SEQ ID NO: 15), and a peak with an area ratio of 88.25% at a retention time of 15.469 minutes was given by HPLC analysis for the synthetic peptide. Alternatively, [M+1] m/z 1540.6 (calculated value: m/z 1538.7) was given by mass spectrometry for the synthetic peptide containing the amino acid sequence of SEQ ID NO: 6 (SEQ ID NO: 16), and a peak with an area ratio of 76.47% at a retention time of 12.524 minutes was observed by HPLC analysis for the synthetic peptide. The synthetic peptide was coupled to keyhole limpet hemocyanin (KLH) via a spacer, so as to prepare a conjugated peptide. Sulfo-SMCC (Sulfosuccinimidyl-4-[N-maleimidomethyl]cyclohexane-1-carbox ylate; Pierce) was used as the spacer. Synthesis of the peptides and preparation of the KLH-conjugated peptides were entrusted to Thermo ELECTRON.

For use in immunization, the KLH-conjugated peptide was dissolved in an 8 M urea solution, resulting in a concentration of 100 µg/ml. In an immunization method, a male BN rat (purchased from Charles River Laboratories, Japan) or female New Zealand white rabbit (purchased from Charles River Laboratories, Japan) was subcutaneously or intramuscularly immunized with 6 shots in total, the first shot in combination with a complete Freund adjuvant and the subsequent shots in combination with an incomplete adjuvant, at 2-week intervals. For such immunization, 20 µg/animal each of the formalin-inactivated strain, the PA5158 recombinant protein, and the KLH-conjugated peptides described in Example 5 was used. One week after the final immunization, whole blood was collected from the abdominal aorta or carotid artery, and it was left at room temperature for 1 hour and centrifuged at 1500 G for 20 minutes, so as to obtain a supernatant having approximately 5 ml/rat or 50 ml/rabbit of sera.

### Example 6: Purification of the IgG fraction from antisera

An IgG fraction was purified from the rat and rabbit antisera according to, for example, the method of McCauley R & Racker, E; Molecular and Cellular Biochemistry 1, 73-81 (1973). An ice-cold saturated ammonium sulfate solution (pH 8) was added to prepare a 43 (v/v)% suspension, and the obtained suspension was stirred at room temperature for 15 minutes. Precipitates were collected by centrifugation at 10,000xg for 20 minutes and dissolved in a 10 mM potassium phosphate buffer (pH 8) supplemented with 10% glycerol. Then, precipitates were deposited again by addition of an ice-cold saturated ammonium sulfate solution (pH 8) to prepare a 50 (v/v)% solution, so as to complete 2 washes. The precipitates were dissolved in a 10 mM potassium phosphate buffer (pH 8) supplemented with 10% glycerol, and subsequently dialyzed overnight against the buffer. The dialysate was centrifuged and then applied to cation-exchange chromatography (DEAE-Toyopearl 650M (Tosoh)). The pass-through volume was collected as an IgG fraction by measuring ultraviolet absorption at 280 nm. The collected fraction was concentrated using Amicon Ultra-15 (Millipore), and the buffer was finally exchanged with a PBS (-) solution, so as to obtain a final sample. 3.0 to 8.0 mg and 30 to 50 mg of proteins were obtained as IgG fractions by purification from 3 ml of the rat antisera and 15 ml of the rabbit antisera, respectively. The proteins were quantified according to DC Protein Assay (Bio-Rad) based on the Lowry method, and IgG purity was evaluated by SDS-PAGE.

As an alternative method, Protein G affinity column chromatography was used in purification of an IgG fraction from the rabbit antisera. The method was carried out according to instructions included with HiTrap Protein G HP (Amersham). The rabbit antisera were added to HiTrap Protein G HP column equilibrated with a 20 mM sodium phosphate buffer (pH 7), and the column was washed with the same buffer. Elution was carried out using a 0.1 M glycine-HCl buffer (pH 2.7), and the eluate was immediately neutralized with a 1/20 volume of a 1.0 M tris-HCl buffer (pH 9) placed in a fractionation tube in advance. A volume to be collected was determined by measuring ultraviolet absorption at 280 nm. The collected fraction was concentrated using Amicon Ultra-15 (Millipore), and the buffer was finally exchanged with a PBS (-) solution, so as to obtain a final sample. Approximately 60 mg of proteins was obtained as an IgG fraction by purification from 10 ml of the rabbit antisera. The proteins were quantified according to DC Protein Assay (Bio-Rad) based on the Lowry method, and IgG purity was evaluated by SDS-PAGE.

### Example 7: Whole cell ELISA test

For whole cell ELISA, a bacterial suspension of PA103 strains cultured in an LB medium was dispensed into an ELISA plate (MaxiSorp Type, NUNC), followed by immobilization at 4°C. The plate was then washed with TBS containing 0.05% Tween 20 and blocked with TBS containing 2% bovine serum albumin and 0.05% Tween 20. Thereafter, the sera or purified IgG fraction obtained in Example 5 were diluted with a saline and added as a primary antibody sample to the plate, and this sample was allowed to react with the strains at 37°C for 1 hour. After washing, peroxidase-labeled goat anti-rat IgG antibody (5000 fold diluted, Sigma) or peroxidase-labeled goat anti-rabbit IgG antibody (5000 fold diluted, Sigma) was used as a secondary antibody, and an enzyme reaction was carried out by addition of a chromogenic substrate (TMB Microwell Peroxidase substrate System, KPL) and then terminated with 0.18 M sulfuric acid. Absorbance at 450 nm was measured.

As a result, with regard to the PA5158 recombinant protein-immunized rat sera, the absorbance of negative control rat sera before immunization (100 fold diluted) was 0.031, whereas the absorbance of the PA5158 recombinant protein-immunized rat sera (100 fold diluted) was 0.399. This indicates that the antibody (IgG) that recognizes the bacterial cell surface-exposed extracellular region of the PA5158 protein is contained in the PA5158 recombinant protein-immunized rat sera.

Moreover, with regard to the purified IgG fraction obtained from the PA5158 recombinant protein-immunized rabbit sera, the absorbance of an IgG fraction (5 µg/ml) purified from negative control rabbit sham sera was 0.15, whereas the absorbance of the IgG fraction (5 µg/ml) purified from the PA5158 recombinant protein-immunized rabbit sera was 0.990. This indicates that the antibody (IgG) that recognizes the cell surface-exposed extracellular region of the PA5158 protein was contained in the IgG fraction derived from the PA5158 recombinant protein-immunized rabbit sera.

Furthermore, with regard to the purified IgG fraction obtained from the PA5158 recombinant protein-immunized rat sera, the absorbance of an IgG fraction (50 µg/ml) purified from sham-operated rat sera as negative control was 0.132, whereas the absorbance of the IgG fraction (50 µg/ml) purified from the PA5158 recombinant protein-immunized rat sera was 0.370. Moreover, the absorbance of the IgG fraction (50 µg/ml) purified from the sera of the rat immunized with the KLH-conjugated peptide of SEQ ID NO: 16 was 0.322. This indicates that the antibody (IgG) that recognizes the cell surface-exposed extracellular region of the PA5158 protein was contained in the IgG fractions derived from the PA5158 recombinant protein-immunized rat sera and the KLH-conjugated peptide-immunized rat sera.

Under the same conditions, whole cell ELISA was carried out using E. *coli* (ATCC25922), which is also a Gram-negative bacterium as with *Pseudomonas aeruginosa.* As a result, the absorbance of an IgG fraction (50 µg/ml) purified from sham-operated rat sera as negative control was 0.118, whereas the absorbance of an IgG fraction (50 µg/ml) purified from the PA5158 recombinant protein-immunized rat sera was 0.090. Moreover, the absorbance of an IgG fraction (50 µg/ml) purified from the sera of the rat immunized with the KLH-conjugated peptide of SEQ ID NO: 16 was 0.072. This indicates that the antibody (IgG) that recognizes an *E*. *coli* cell surface-exposed antigen was not contained in the IgG fractions derived from the PA5158 recombinant protein-immunized rat sera and the KLH-conjugated peptide-immunized rat sera. Thus, this result indicates that these IgG fractions can be used in the detection of *Pseudomonas aeruginosa.*

### Example 8: ELISA test

### (1) Detection of antibody that binds to PA5158 recombinant protein

In order to detect an antibody that binds to the PA5158 recombinant protein by the ELISA method, the PA5158 recombinant protein was dissolved in PBS supplemented with 8 M urea, and placed at a concentration of 0.5 µg/well of the protein in a 96-well nickel plate (HIS-Select High Sensitivity (HS) Nickel Coated Plates, Sigma). The plate was left at room temperature for 1 hour, so as to cause binding of the protein to the plate. The plate was washed with a wash buffer (PBS supplemented with 0.05% Tween 20, 5 mM imidazole, and 500 mM NaCl) and blocked with a blocking buffer (wash buffer supplemented with 0.5% gelatin). Then, the antibody-containing sample obtained in Example 5 was added to the wells and allowed to react for 30 minutes, and the plate was then washed. A secondary antibody (peroxidase-labeled goat anti-rat IgG antibody, 10000 fold diluted, Sigma) was added thereto and allowed to react therewith for 30 minutes, and the plate was then washed. An enzyme reaction was carried out by addition of a chromogenic substrate (TMB Microwell Peroxidase Substrate System, KPL) and then terminated with 1 M phosphoric acid. Absorbance at 450 nm was measured.

As a result, the absorbance of negative control sera before immunization (10,000 fold diluted) was 0.058, whereas the absorbance of the PA5158 recombinant protein-immunized rat sera (10,000 fold diluted) was 0.410. This indicates that the antibody that binds to the PA5158 recombinant protein as an immunogen is contained in the PA5158 recombinant protein-immunized rat sera.

Moreover, the absorbance of negative control rat sham IgG (10 µg/ml) purified from sera obtained from rat to which only an adjuvant had been administered was 0.190, whereas the absorbance of rat anti-PA5158 IgG (10 µg/ml) as the IgG fraction purified from the PA5158 recombinant protein-immunized rat sera was 2.918. This indicates that the antibody that binds to the PA5158 recombinant protein as an immunogen is contained in the rat anti-PA5158 IgG.

### (2) Detection of antibody that binds to putative extracellular region peptide of the PA5158 protein

In order to detect an antibody that binds to the putative extracellular region peptide (SEQ ID NO: 5 or SEQ ID NO: 6) of the PA5158 protein by ELISA, each peptide synthesized in Example 5 was dissolved in a carbonate buffer (0.15% Na₂CO₃ and 0.3% NaHC0₃) and placed at a concentration of 1 or 2 µg/well of the peptide in a 96-well plate (MaxiSorp, Nunc). The plate was left overnight at 4°C so as to cause adsorption of the peptide onto the plate. The plate was washed with PBS and blocked with PBS supplemented with 0.5% bovine serum albumin. Then, the antibody-containing sample obtained in Example 5 was added to the wells after dilution and allowed to react with the peptide for 1 hour, and the plate was then washed with PBS containing 0.05% Tween 20. A secondary antibody was added to the wells and allowed to react therewith for 30 minutes, and the plate was then washed with PBS containing 0.05% Tween 20. A chromogenic reaction and measurement of absorbance were carried out in the same way as above.

As a result, the absorbance of negative control sera before immunization (100 fold diluted) was 0.053 in the well coated with 1 µg of the putative extracellular region peptide (SEQ ID NO: 5) of the PA5158 protein, whereas the absorbance of the PA5158 recombinant protein-immunized rat sera (100 fold diluted) was 0.445 in such a well. This indicates that the antibody that binds to the extracellular peptide (SEQ ID NO: 5) of the PA5158 protein is contained in the PA5158 recombinant protein-immunized rat sera.

Moreover, the absorbance of negative control rat sham IgG (10 µg/ml) as an IgG fraction purified from the sera of a rat to which only an adjuvant had been administered was 0.135 in the well coated with 2 µg of the putative extracellular region peptide (SEQ ID NO: 5) of the PA5158 protein, whereas the absorbance of rat anti-PA5158 IgG (10 µg/ml) as the IgG fraction purified from the PA5158 recombinant protein-immunized rat sera was 0.368 in such well. This indicates that the antibody that binds to the extracellular peptide (SEQ ID NO: 5) of the PA5158 protein is contained in the rat anti-PA5158 IgG.

Furthermore, in such detection of the antibody that binds to the putative extracellular region peptide (SEQ ID NO: 6) of the PA5158 protein, the peptide in which 6 amino acids were added to the N-terminal (SEQ ID NO: 17) for improving the solubility of the protein was used to coat the wells with 2 µg/well of the peptide.

As a result, the absorbance of negative control rat sham IgG (100 µg/ml) as an IgG fraction purified from the sera of a rat to which only an adjuvant had been administered was 0.160, whereas the absorbance of rat anti-PA5158 IgG (100 µg/ml) as the IgG fraction purified from the PA5158 recombinant protein-immunized rat sera was 0.589. This indicates that the antibody that binds to the extracellular peptide (SEQ ID NO: 6) of the PA5158 protein is contained in the rat anti-PA5158 IgG.

### Example 9: Ability of PA5158 recombinant protein-immunized rat sera to defend against PA103 strain infection

Viable PA103 strains suspended in 100 µl of a saline containing 5% mucin were intraperitoneally administered at a dose of 1.0×10⁵ cfu/mouse (20 LD₅₀) to 4-week-old CD-1 mice (purchased from Charles River Laboratories, Japan). Immediately thereafter, the PA5158 recombinant protein-immunized rat sera (all serum samples were 2.5 fold diluted with a saline) were administered at a dose of 10 ml/kg from the caudal vein. Seven days later, protective activity against the infection was determined based on survival.

As a result, 5 out of 7 negative control sham rat serum-administered mice died, whereas all mice survived in a formalin-inactivated PA103 strain-immunized rabbit serum-administered group. It was thus confirmed that the formalin-inactivated PA103 strain-immunized rabbit serum has protective activity against the infection. Under this condition, 6 out of 7 mice survived in the PA5158 recombinant protein-immunized rat serum-administered group. It was thus confirmed that the PA5158 recombinant protein-immunized rat serum has protective activity against the infection.

### Example 10: Ability of PA5158 recombinant protein-immunized rat sera to defend against PA103 strain infection in neutropenic mice

12.5 mg/ml (saline) of Cyclophosphamide monohydrate (hereinafter referred to as CY, Sigma-Aldrich) was prepared and intraperitoneally administered at a dose of 125 mg/kg to 4-week-old CD-1 mice on day -5, -2, and 0, so as to reduce a neutrophil level in peripheral blood. Then, viable PA103 strains were intraperitoneally administered at a dose of 1.83×10⁵ cfu/mouse (136 LD₅₀) to the mice. Immediately thereafter, the PA5158 recombinant protein-immunized rat sera (all serum samples were 2.5 fold diluted with a saline) were administered at a dose of 10 ml/kg from the caudal vein. Seven days later, protective activity against the infection was determined based on survival.

As a result, all of 7 negative control sham rat serum-administered mice died, whereas 4 out of 7 mice survived in a formalin-inactivated PA103 strain-immunized rat serum-administered group. It was thus confirmed that the formalin-inactivated PA103 strain-immunized rat serum has protective activity against the infection. Under this condition, 3 out of 7 mice survived in the PA5158 recombinant protein-immunized rat serum-administered group. It was thus confirmed that the PA5158 recombinant protein-immunized rat serum has protective activity against the infection.

### Example 11: Ability of PA5158 recombinant protein-immunized rat sera to defend against multidrug resistant Pseudomonas aeruginosa infection in neutropenic mice

The minimum inhibitory concentrations of various types of antimicrobial agents for multidrug resistant *Pseudomonas aeruginosa* MSC06120 strains were imipenem: 32 µg/ml, amikacin: 64 µg/ml, and ciprofloxacin: >256 µg/ml. 12.5 mg/ml (saline) of CY was prepared and intraperitoneally administered at a dose of 125 mg/kg to 4-week-old CD-1 mice on day -5, -2, and 0, so as to reduce a neutrophil level in peripheral blood. Then, viable MSC06120 strains were intraperitoneally administered at a dose of 1.23×10⁵ cfu/mouse (11 LD₅₀) to the mice. Immediately thereafter, the PA5158 recombinant protein-immunized rat sera (all serum samples were 2.5 fold diluted with a saline) were administered at a dose of 10 ml/kg from the caudal vein. Seven days later, protective activity against the infection was determined based on survival.

As a result, 5 out of 7 negative control sham rat serum-administered mice died, whereas 6 out of 7 mice survived in a formalin-inactivated PA103 strain-immunized rat serum-administered group. It was thus confirmed that the formalin-inactivated PA103 strain-immunized rat serum has protective activity against the infection. Under this condition, 6 out of 7 mice survived in the PA5158 recombinant protein-immunized rat serum-administered group. It was thus confirmed that the PA5158 recombinant protein-immunized rat serum has protective activity against the infection.

### Example 12: Ability of rat anti-PA5158 antibody to defend against multidrug resistant Pseudomonas aeruginosa infection in neutropenic mice

Viable MSC06120 strains were intraperitoneally administered at a dose of 1.18×10⁵ cfu/mouse (10 LD₅₀) to mice. Immediately thereafter, the rat anti-PA5158 IgG as the IgG fraction purified from the PA5158 recombinant protein-immunized rat sera was administered at a dose of 1 mg/mouse from the caudal vein. Seven days later, protective activity against the infection was determined based on survival.

As a result, 4 out of 7 mice died in a negative control sham rat IgG (1 mg/mouse)-administered group, whereas all of 7 mice survived in a group to which rat anti-PA103 IgG as an IgG fraction purified from the sera of a rat immunized with formalin-inactivated PA103 strains had been administered. It was thus confirmed that the rat anti-PA103 IgG has protective activity against the infection. Under this condition, 6 out of 7 mice survived in the group to which the rat anti-PA5158 IgG as the IgG fraction purified from the PA5158 recombinant protein-immunized rat sera had been administered. It was thus confirmed that the rat anti-PA5158 IgG has protective activity against the infection.

### Example 13: Preparation of monoclonal antibody (MAb)

One week after the final immunization with the prepared PA5158 recombinant protein in Example 5, the spleen was aseptically extracted under anesthesia. The obtained spleen was washed with an RPMI-1640 medium (Gibco), and it was then inserted between slide glasses and crushed, so as to obtain a splenic cell test sample as fine small pieces. The obtained splenic cells were washed by centrifugation at 1200 rpm for 5 minutes using an RPMI-1640 medium. On the other hand, myeloma cells (P3X63Ag8U1 cells) were cultured in advance under conditions of 5% CO₂, relative humidity of 100%, and 37°C in an RPMI-1640 medium containing 10% FCS (fetal bovine serum), and the thus cultured myeloma cells during the exponential phase of growth were washed by centrifugation using an RPMI-1640 medium and mixed with the aforementioned splenic cells, such that a ratio of the number of the splenic cells to the number of the myeloma cells became approximately 4:1. The mixed cells were centrifuged at 1000 rpm for 10 minutes. The supernatant was discarded, and the cells were sufficiently loosened. To the centrifuge tube containing the cells, 1 mL of a solution consisting of 2 g of polyethylene glycol (M.W. 1000, Wako Pure Chemical Industries), 2 mL of an RPMI-1640 medium, and 0.2 mL of DMSO (Nacalai Tesque) was gently added. The cells were mixed by slowly rotating the centrifuge. One minute later, the centrifuge tube was slowly rotated, while 15 mL of an RPMI-1640 medium was added thereto over 3 minutes. The cells were centrifuged at 1000 rpm for 7 minutes. Then, the supernatant was discarded, and the cells were sufficiently loosened. Thereafter, the cell concentration was adjusted to 1.6×10⁶ cells/mL in terms of splenic cells using a HAT medium (Gibco). The resulting cells were dispensed at a concentration of 0.2 mL/well into a 96-well microplate (Sumitomo Bakelite). The cells were cultured under conditions of 5% CO₂, relative humidity of 100%, and 37°C. Approximately 1 to 2 weeks later, hybridomas grown in the wells were observed under a microscope.

### (1) Screening of antibody of interest

An antibody that binds to the cell surface of *Pseudomonas aeruginosa* was detected by the whole cell ELISA described in Example 7. Moreover, an antibody that binds to the PA5158 recombinant protein or the putative extracellular region (SEQ ID NO: 5 or SEQ ID NO: 6) of the PA5158 protein was detected by the ELISA described in Example 8.

### (2) Cloning of cells producing antibody of interest

The concentration of BALB/c mouse thymic cells was adjusted to 2×10⁷ cells/mL using a 10% FCS/HT medium. The resulting cells were dispensed at a concentration of 0.1 mL/well into a 96-well microplate (Sumitomo Bakelite). The cells were cultured overnight under conditions of 5% CO₂, relative humidity of 100%, and 37°C. On the following day, the hybridomas determined by screening to produce the antibody of interest were adjusted to 5 hybridomas/0.1 mL using a 10% FCS HT (Gibco) medium, and were dispensed at a concentration of 0.1 mL/well to the wells in which the thymic cells were dispensed on the preceding day. One to two weeks later, the growth of clones could be observed under a microscope. The clones were analyzed by the method described in the paragraph for screening, so as to select clones producing the antibody of interest. The hybridomas having a concentration adjusted to 1 hybridoma/0.1 mL using a 10% FCS/HT (Gibco) medium by the aforementioned method were dispensed at a concentration of 0.1 mL/well to the wells in which the thymic cells were dispensed on the preceding day. One to two weeks later, clones were analyzed by the method described in the paragraph for screening, so as select monoclones producing the antibody of interest.

### (3) In vitro culture of cells and production of MAb

The clones of interest sufficiently proliferated in the 96-well microplate were scaled up in stages in a 24-well plate, a 50-mL flask, and a 250-mL flask and cultured in a 10% FCS-RPMI medium. MAb produced in the culture supernatant of the cells thus obtained was detected by the ELISA described in Example 8.

This ELISA was carried out for detecting binding to the well on which the synthetic peptide (SEQ ID NO: 15) of Example 5, which contained the extracellular region of SEQ ID NO: 5 estimated based on three-dimensional structure information about a *Pseudomonas aeruginosa* PA0427 (OprM) protein (J. Biol. Chem., 2004, 279, 52816-52819), three-dimensional structure information about an *E*. *coli* ToIC protein (Nature, 2000, 405, 914-919), and secondary structure information about the PA5158 protein, had been absorbed. As a result, in such ELISA, the absorbance was 0.053 in a 10% FCS-RPMI medium as a negative control, whereas the absorbance was 0.903 in the culture supernatant of the hybridoma deposited with the National Institute of Advanced Industrial Science and Technology, International Patent Organism Depositary under the accession No. FERM BP-10672. It was therefore revealed that the MAb that binds to the peptide is produced.

### Example 14: Enhancement of anti-Pseudomonas aeruginosa activity of an aminoglycoside antibiotic

It has been reported that the PA5158 protein is an outer membrane protein that constitutes efflux pumps directly involved in resistance to aminoglycoside antibiotics (Antimicrobial Agents and Chemotherapy, 2003, 47, 1101-1111). Variants deficient in this protein possess increased sensitivity to aminoglycoside antibiotics. Thus, whether or not various types of antibodies that recognize this protein obtained by the present inventors have an inhibitory effect on aminoglycoside antibiotic efflux pumps was tested.

*Pseudomonas aeruginosa* PA103 strains (ATCC29260) were shake cultured overnight at 37°C in a Mueller-Hinton liquid medium and diluted to 1×10⁵ cfu/ml with the same medium. Then, the strains were shake cultured again at 37°C. Two hours later, gentamicin was added at a concentration of 0.125 µg/ml. Likewise, to a negative control group, PBS was added at a concentration of 300 µg/ml. To antibody-added groups, rat anti-PA5158L1 IgG as the monoclonal antibody produced by the hybridoma deposited under the accession No. FERM-BP-10672, rat anti-PA5158L2 IgG as the IgG fraction purified from the KLH-conjugated peptide (SEQ ID No: 6)-immunized rat sera, and rat anti-PA5158 IgG as the IgG fraction purified from the PA5158 recombinant protein-immunized rat sera were added at each concentration of 300 µg/ml. Then, shake culture was carried out at 37°C for 4 hours, and the number of viable strains in each group was measured.

As a result, the strains were proliferated to 1.05×10⁸ cfu/ml in the control group, whereas the strains were proliferated to 5.4×10⁶ cfu/ml in the negative control group. Furthermore, the strains were proliferated to 6.4×10⁵ cfu/ml, 7.5×10⁵ cfu/ml, and 6.3×10⁵ cfu/ml, respectively, in the antibody-added groups, all of which were lower than the number of viable strains in the negative control group. It was thus confirmed that each of these antibodies has an effect of enhancing antimicrobial activity of an aminoglycoside antibiotic gentamicin.

All of the antibodies have a property of being capable of binding to the PA5158 protein that constitutes aminoglycoside antibiotic efflux pumps, and a portion of the sequence thereof. It was therefore estimated that the present effect of enhancing anti-*Pseudomonas aeruginosa* activity of aminoglycoside antibiotic results from inhibition of the efflux pumps.

## Claims

1. An antigen composition comprising a protein antigen or a peptide antigen which can induce the production of an antibody against a *Pseudomonas aeruginosa* PA5158 protein.

2. A protein selected from the group consisting of:
(i) a protein comprising the amino acid sequence of SEQ ID NO: 4;
(ii) a protein which comprises an amino acid sequence of SEQ ID NO: 4 in which one or more amino acids are deleted, substituted, inserted, or added, and which is functionally equivalent to a protein consisting of the amino acid sequence of SEQ ID NO:4;
(iii) a protein which is encoded by a polynucleotide which hybridizes under stringent conditions to a polynucleotide which encodes the amino acid sequence of SEQ ID NO: 4, and which is functionally equivalent to a protein consisting of the amino acid sequence of SEQ ID NO:4; and
(iv) a protein which comprises an amino acid sequence having 70% or more identity with the amino acid sequence of SEQ ID NO: 4, and which is functionally equivalent to a protein consisting of the amino acid sequence of SEQ ID NO:4.

3. A peptide comprising the amino acid sequence of SEQ ID NO: 5, or an amino acid sequence of SEQ ID NO: 5 which contains one to several conservative substitutions.

4. A peptide comprising the amino acid sequence of SEQ ID NO: 6, or an amino acid sequence of SEQ ID NO: 6 which contains one to several conservative substitutions.

5. An antigen composition comprising a protein according to claim 2, or a peptide according to claim 3 or 4.

6. A vaccine composition for use in the prevention or treatment of diseases associated with *Pseudomonas aeruginosa,* comprising an antigen composition according to claim 1 or 5, and optionally one or more pharmaceutically acceptable carriers, diluents, and/or adjuvants.

7. An antibody against a *Pseudomonas aeruginosa* PA5158 protein or a portion thereof, or a functional fragment thereof.

8. An antibody or a functional fragment thereof according to claim 7, wherein the portion of the *Pseudomonas aeruginosa* PA5158 protein is a cell surface-exposed portion of the *Pseudomonas aeruginosa* PA5158 protein.

9. An antibody or a functional fragment thereof according to claim 8, wherein the cell surface-exposed portion of the *Pseudomonas aeruginosa* PA5158 protein is selected from the group consisting of:
(i) a protein comprising the amino acid sequence of SEQ ID NO: 4;
(ii) a protein which comprises an amino acid sequence of SEQ ID NO: 4 in which one or more amino acids are deleted, substituted, inserted, or added, and which is functionally equivalent to a protein consisting of the amino acid sequence of SEQ ID NO:4;
(iii) a protein which is encoded by a polynucleotide which hybridizes under stringent conditions to a polynucleotide which encodes the amino acid sequence of SEQ ID NO: 4, and which is functionally equivalent to a protein consisting of the amino acid sequence of SEQ ID NO:4; and
(iv) a protein which comprises an amino acid sequence having 70% or more identity with the amino acid sequence of SEQ ID NO: 4, and which is functionally equivalent to a protein consisting of the amino acid sequence of SEQ ID NO:4.

10. An antibody or a functional fragment thereof according to claim 8, wherein the cell surface-exposed portion of the *Pseudomonas aeruginosa* PA5158 protein is a peptide comprising the amino acid sequence of SEQ ID NO: 5, or an amino acid sequence of SEQ ID NO: 5 which contains one to several conservative substitutions.

11. An antibody or a functional fragment thereof according to claim 10, which is produced by a hybridoma deposited under the accession No. FERM BP-10672.

12. An antibody or a functional fragment thereof according to claim 8, wherein the cell surface-exposed portion of the Pseudomonas aeruginosa PA5158 protein is a peptide comprising the amino acid sequence of SEQ ID NO: 6, or an amino acid sequence of SEQ ID NO: 6 which contains one to several conservative substitutions.

13. An antibody or a functional fragment thereof according to any one of claims 7 to 12, wherein the antibody is a monoclonal antibody.

14. A hybridoma deposited under the accession No. FERM BP-10672.

15. An antibody or a functional fragment thereof according to claim 7, which is a monoclonal antibody cross-reactive with the same antigen as that of the monoclonal antibody produced by a hybridoma according to claim 14.

16. A pharmaceutical composition for use in the prevention or treatment of diseases associated with *Pseudomonas aeruginosa,* comprising an antibody or a functional fragment thereof according to any one of claims 7 to 9, and optionally one or more pharmaceutically acceptable carriers and/or diluents.

17. A vaccine composition according to claim 6 or a pharmaceutical composition according to claim 16, wherein the disease associated with Pseudomonas aeruginosa is a systemic infectious disease caused by a *Pseudomonas aeruginosa* infection.

18. A vaccine composition according to claim 6 or a pharmaceutical composition according to claim 16, wherein the *Pseudomonas aeruginosa* infection is a multidrug resistant *Pseudomonas aeruginosa* infection.

19. A diagnostic agent for a *Pseudomonas aeruginosa* infection, comprising an antibody or a functional fragment thereof according to any one of claims 7 to 13, and 15.

20. A kit for detecting *Pseudomonas aeruginosa,* comprising an antibody or a functional fragment thereof according to any one of claims 7 to 13, and 15.

21. An agent for enhancing *anti-Pseudomonas aeruginosa* activity of an aminoglycoside antibiotic, comprising an antibody or a functional fragment thereof according to any one of claims 7 to 13, and 15.
